# EUROPEAN PATENT SPECIFICATION

(11) **EP 2 481 369 B1**
(45) Date of publication and mention of the grant of the patent: **21.10.2015**
(21) Application number: 12153219.6
(22) Date of filing: 31.01.2012
(51) Int. Cl.: A61B 18/12, A61B 18/14

(54) **Prevention of safety hazards due to leakage current**
Vorbeugung von Sicherheitsrisiken aufgrund von Kriechstrom
Prévention des risques pour la sûreté dus à un courant de fuite

(30) Priority: 01.02.2011 US 201113018773
(43) Date of publication of application: 01.08.2012
(73) Proprietor: Biosense Webster (Israel), Ltd., 20692 Yokneam (IL)
(72) Inventor: Levin, Michael, 35590 Haifa (IL); Govari, Assaf, 34400 Haifa (IL); Ephrath, Yaron, 35170 Karkur (IL); Altmann, Andres Claudio, 34757 Haifa (IL)
(74) Representative: Small, Gary James

(56) References cited:
- EP-A1- 0 544 415
- EP-A1- 1 352 615
- DE-A1- 3 824 913
- GB-A- 2 177 309
- US-A- 3 895 635
- US-A- 4 200 105
- US-A- 4 231 372
- US-A- 4 793 345
- US-A- 5 064 994
- US-A- 5 152 762

## Description

### FIELD OF THE INVENTION

The present invention relates generally to medical electronic devices, and specifically to circuits for improving patient safety in such devices.

### BACKGROUND OF THE INVENTION

Medical electronic devices that come into contact with the human body must meet stringent safety requirements. One such requirement is a limitation of the currents passing through the patient's body.

IEC 60601-1 standard, "Medical Electrical Equipment-Part 1: General Requirements for Safety and Essential Performance" (Geneva: International Electrotechnical Commission, 1995), which is incorporated herein by reference, describes tests and limits for leakage current, as do a number of related national standards. These standards relate to three types of the leakage currents: the patient leakage current (PLC), patient auxiliary leakage current (PALC), and patient leakage current by mains voltage on applied part (PLCMVAP). The applied part (AP) of the medical device is considered to include all connections to the patient's body. The PLC is the current that passes between all the AP wires connected together and the protective ground. The PALC is the current that passes between any wires of the AP.

The PLC and PALC currents can exist during normal conditions (NC) of the medical device, as well as during fault conditions (FC). IEC60601-1 limits both of the leakage currents to 10 µA in NC and to 50 µA in single fault conditions (SFC). The PLCMVAP can exist only in FC and is likewise limited to 50 µA. The permitted leakage values depend on the class of medical equipment and the type of AP. The above leakage values are for Class 1 equipment with "cardiac floating" (CF) apply parts, and apply to most cardiological equipment.

US-A-3 895 635 discloses an electrosurgery systems including ECG equipment and a common ECG lead monitor and suggests monitoring the integrity of the common lead's connection to ground by sending a test current through the lead.

### SUMMARY OF THE INVENTION

The invention provides an apparatus according to claim 1. Preferred embodiments are defined in the dependent claims.

There is provided, in accordance with the present invention, medical apparatus, including an electrical power supply, which is configured for connection to an electrical medical device that is coupled to a body of a patient and which includes a power connection. A switch is coupled to connect and disconnect the power connection. A plurality of electrocardiogram (ECG) leads, including a common lead, are coupled to the body of the patient. An auxiliary current sensor is coupled to monitor a current flowing through the common lead and to actuate the switch to disconnect the power connection when the current exceeds a predetermined limit.

Typically, the common lead is connected to a right leg of the patient.

In a disclosed embodiment, the auxiliary current sensor includes a resistor connected between the common lead and a reference point, and a sensing circuit coupled to measure a voltage drop across the resistor.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for medical diagnosis and treatment, in accordance with an embodiment of the present invention;
Fig. 2 is a block diagram that schematically illustrates an ablation energy generator under test, in accordance with an embodiment of the present invention; and
Fig. 3 is a schematic circuit diagram illustrating a circuit for measurement of PALC, in accordance with an embodiment of the present invention.

### DETAILED DESCRIPTION OF EMBODIMENTS

Common safety standards for medical electronic devices define maximum steady-state leakage currents that can be allowed to flow through the patient's body when contacted by a medical device. For example, as noted above, the above-mentioned IEC 60601-1 specifies a maximum patient leakage level of 10 µA in the frequency range up to 1 kHz under normal conditions and 50 µA in single fault conditions. The standard specifies the single fault conditions for which this standard is applicable, such as presence of mains voltage on the applied part. This condition may occur when lines voltage is accidentally applied to the patient (or to the part of the device that contacts the patient's body - PLCMVAP).

To keep the steady-state leakage currents below the specified limits under such conditions, medical device manufacturers typically use well-insulated isolation transformers and other means to separate the part of the device that comes in contact with the patient from the power lines, including isolation of the patient from the power line ground. In high-power devices, such as radio frequency (RF) ablators, isolation transformers of this sort and associated components can be large and costly.

Embodiments of the present invention take a different approach, which enables compliance with leakage standards, particularly with respect to PALC and PLCMVAP, while using less bulky, less costly means of isolation. Devices that operate in accordance with these embodiments meet leakage current requirements in steady state under normal conditions. When a fault condition occurs, however, a sensor detects the related leakage overcurrent and disconnects the device from the mains or other power supply (such as a battery).

These embodiments can be implemented in a wide variety of systems for medical diagnosis and/or treatment, which typically include an electrical power supply connected to a medical device that comes in contact with the body of a patient. For example, the power supply may be part of a RF generator that feeds an electrode on a catheter for the purpose of RF ablation. In this sort of equipment (at least in Class 1), the power supply typically has an alternating current (AC) lines connection that includes a ground line.

In a disclosed embodiment, the power supply is connected to the AC lines via a high-speed safety switch, such as a high-power relay. To prevent PLCMVAP overcurrent, a sensitive current sensor monitors the current on the ground line, which should be close to zero under normal conditions. If and when the sensor detects a ground current that exceeds a certain threshold, such as a level approaching or equal to the limit imposed an by applicable standard (as may occur, for example, when a high voltage is applied to the patient or to the catheter electrode), the sensor immediately actuates the switch to disconnect the AC lines, thus disabling the ablator and breaking the connection between the catheter and lines ground.

This approach makes it possible to reduce the level of steady-state isolation between the patient and the lines ground in the medical system, and thus to use smaller and less costly isolation components, which still give adequate isolation under normal operating conditions. The sensor and switch take over to protect against increased leakage under fault conditions. Although this approach is unconventional, it protects the patient sufficiently and complies with known safety standards. It is particularly beneficial in reducing the cost and size of high-power medical devices, such as ablators.

Alternatively or additionally, PALC in the medical system may be monitored by directly measuring electrical current flowing through the patient's body. In many systems, electrocardiogram (ECG) leads are coupled to the body for monitoring purposes. The lead set typically includes several leads connected to high-impedance inputs of respective amplifiers and a common lead connected to an isolated common point or isolated ground of the amplifiers. The common lead is conventionally attached to the patient's right leg (but may be attached elsewhere on the body). In a disclosed embodiment, an additional current sensor monitors the PALC current flowing through the common lead and actuates the switch to disconnect the AC lines or battery if and when the current exceeds a predetermined limit.

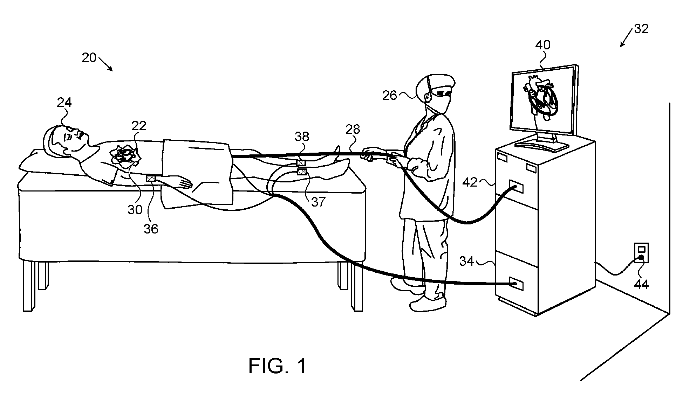

Fig. 1 is a schematic, pictorial illustration of a system 20 for cardiac diagnosis and treatment, in accordance with an embodiment of the present invention. System 20 may be based, for example, on the CARTO^{™} system, produced by Biosense Webster Inc. (Diamond Bar, California). System 20 comprises a catheter 28, which is used by an operator 26, typically a cardiologist, in mapping and ablation of tissue in a heart 22 of a patient 24. Operator 26 inserts catheter 28 through the vascular system of patient 24 so that a distal end 30 of the catheter enters a chamber of the heart. The operator advances and manipulates the catheter in the body so that the distal tip of the catheter engages endocardial tissue in the heart chamber in order to sense electrophysiological signals and, as appropriate, to ablate tissue at desired locations.

Catheter 28 is connected at its proximal end to a console 32, comprising a signal processing unit 34, which receives and analyzes signals output by the catheter. In addition, unit 34 receives ECG signals from leads 36, 37, 38, ..., that are attached to the patient's body surface. Typically, these leads comprise adhesive skin electrodes, although leads of other types known in the art may similarly be used. One of the leads, such as lead 37 on the right leg of patient 24, is identified as the common lead and is coupled to a reference point corresponding to the ground potential.

Based on the signals received from catheter 28, electrodes 36, 37, 38, ..., and other components of system 20, processing unit 34 drives a display 40. The display may present information such as a map of heart 22, ECG traces, system messages to operator 26, and any other relevant types of data that are known in the art.

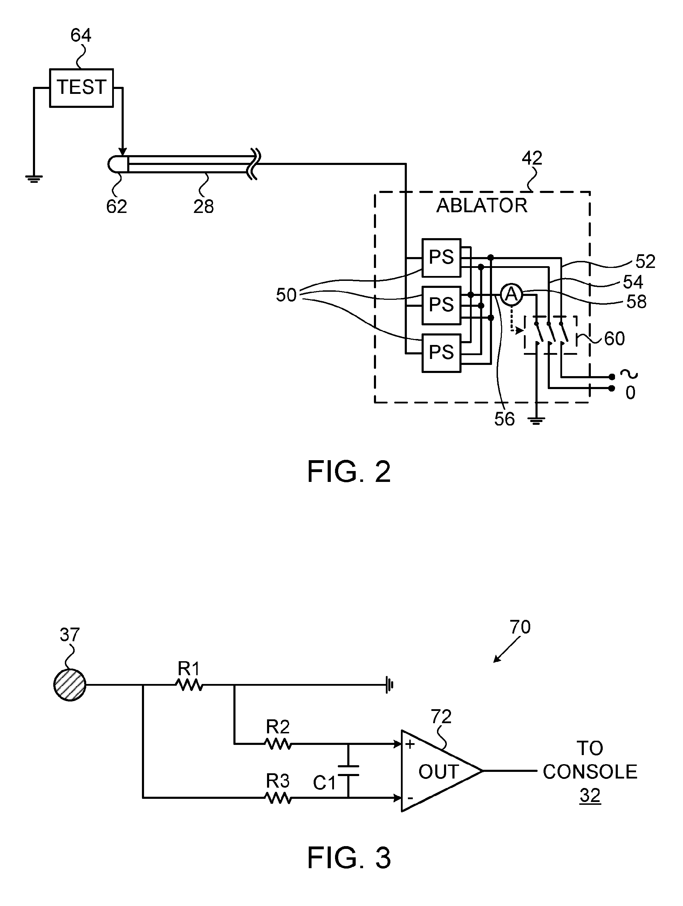

Console 32 also comprises an energy generator 42, which provides energy to catheter 28 for ablating pathological sites in heart 22. For example, energy generator 42 may provide radio frequency (RF) energy to an electrode (shown in Fig. 2) at the distal tip of catheter 28 for performing RF ablation. AC mains power, the power source for system 20, is supplied to energy generator 42 and other components of the system via a lines connector 44.

Fig. 2 is a block diagram that schematically illustrates ablation energy generator 42 under test, in accordance with an embodiment of the present invention. Whereas conventional ablation power supplies are large and costly and must generally be custom-designed to provide high output power with adequate patient isolation, smaller, inexpensive power supplies for medical applications are widely available. Therefore, energy generator 42 comprises a number of such smaller power supplies 50, which are ganged in parallel, in order to supply sufficient power to an ablation electrode 62 at the distal end of catheter 28. These smaller power supplies may individually meet medical safety standards in steady state, but when the power supplies are deployed in parallel, and lines voltage is applied to the patient, the total leakage current through all the power supplies may exceed the standard limit for fault conditions.

To solve this problem, the AC lines, including power lines 52 and 54 and ground line 56, of power supplies 50 are connected to mains power (via connector 44) through a safety switch 60, such as a high-speed, high-power relay. A current sensor 58 is coupled to ground line 56 in order to sense the current flowing to the lines ground. Sensor 58 may comprise, for example, a bandpass filter, to selectively pass and amplify currents in the range of the lines frequency (50-60 Hz), and a RMS-to-DC converter, to generate a measurement of the AC current. If the measured current exceeds a predetermined limit, such as 50 µA under fault conditions, the sensor instructs switch 60 to open and thus disconnect lines 52, 54 and 56 from connector 44, and hence from the mains power and ground. This arrangement of the sensor and switch is thus useful particularly in satisfying safety standards with respect to PLCMVAP.

In safety testing of system 20, a test jig 64 applies lines voltage (for example, 220 volts) to electrode 62, simulating a situation in which this voltage is applied to the patient. Under these circumstances, current sensor 58 will typically sense an increase in leakage current on ground line 56 and, if the current passes a predetermined limit, will immediately open switch 60. Typically, the switch opens within 30 ms.

Fig. 3 is a schematic circuit diagram illustrating a circuit 70 for measurement of PALC, in accordance with an embodiment of the present invention. This circuit may, for example, be integrated into the front end of processing unit 34, or it may be coupled in or to console 32 as a separate unit. Circuit 70 provides a solution to safety problems due to excessive PALC that may be caused by fault conditions in the ECG circuits in system 20. This solution may be used in conjunction with or independently of the PLCMVAP detection and prevention circuits described above.

One way to prevent the patient auxiliary leakage current through common ECG lead 37 from exceeding safety limits is to connect a large serial resistance, typically on the order of 5 MΩ, in series with the lead. A problem with this approach, however, is that the resistor generates thermal noise in the ECG measurement circuits, which is directly proportional to the resistance.

To avoid this noise problem while still preventing excessive leakage under fault conditions, circuit 70 continuously measures the current passing through common lead 37. In circuit 70, lead 37 is connected to the reference point (marked as ground in Fig. 3) through a resistor R1, typically with a small resistance, such as 1 kΩ. A differential amplifier 72, with inputs coupled across this resistor through an input low-pass filter, which can be implemented, for example, by resistors R2 and R3 and a capacitor C1. Typically, the resistances of R1 and R2 are in a range between a few kΩ and several hundred kΩ, and the capacitance of C1 is chosen to give a cut-off frequency of several hundred Hertz. This low pass filter decreases the sensitivity of circuit 70 to noise and thus prevents false alarms.

Differential amplifier 72 provides an output to console 32 that is proportional to the voltage drop across resistor R1. The output of amplifier 72 is thus directly proportional to the patient auxiliary leakage current. This output may be pre-calibrated to find the constant of proportionality and set a safety threshold corresponding to the leakage limit. When the output of amplifier 72 exceeds the threshold, lines connector 44 (or any other power connection that feeds system 20) is automatically disconnected, and system 20 is shut down.

Although the above embodiments are described, for the sake of clarity, with reference to the components of system 20, the principles of the present invention may equally be applied in substantially any other suitable type of electrical medical device. It will be appreciated that the embodiments described above are cited by way of example, and that the present invention is not limited to what has been particularly shown and described hereinabove.

## Claims

1. Medical apparatus, comprising:
an electrical power supply (42), which is configured for connection to an electrical medical device (28) that is coupled to a body of a patient and which comprises a power connection;
a switch (60), which is coupled to connect and disconnect the power connection;
a plurality of electrocardiogram (ECG) leads (36, 37, 38), including a common lead (37), for coupling to the body of the patient; and
an auxiliary current sensor, which is coupled to monitor a current flowing through the common lead (37) and to actuate the switch to disconnect the power connection when the current exceeds a predetermined limit.

2. The apparatus according to claim 1, wherein the common lead (37) is connectable to a right leg of the patient.

3. The apparatus according to claim 1, wherein the auxiliary current sensor comprises a resistor connected between the common lead and a reference point, and a sensing circuit (70) coupled to measure a voltage drop across the resistor.

4. The apparatus according to claim 1, further comprising a current sensor, which is coupled to sense a current flowing in a ground line of the power connection and to actuate the switch to disconnect the power connection when the current exceeds a predetermined threshold.

5. The apparatus according to claim 4, wherein the electrical power supply (42) comprises a plurality of power supplies (50) arranged in parallel to supply power to the medical device.

6. The apparatus according to claim 5, wherein the plurality of the power supplies are configured to supply radio frequency (RF) power to an electrode on the medical device (28) for ablation of tissue from the body.

7. The apparatus according to claim 5, wherein each of the plurality of the power supplies (50) individually has a level of isolation sufficient so that a leakage current in the ground line due to application of an AC lines voltage to the medical device will be less than a limit provided by the IEC 60601-1 standard, while the sum of the leakage current over all of the power supplies exceeds the limit.

## Patentansprüche

1. Medizinischer Apparat, umfassend:
eine elektrische Stromversorgung (42), die für eine Verbindung mit einer elektrischen medizinischen Vorrichtung (28) ausgelegt ist, welche an einen Körper eines Patienten angeschlossen ist und welche einen Stromanschluss umfasst;
einen Schalter (60), der zum Verbinden und Unterbrechen des Stromanschlusses angeschlossen ist;
mehrere Elektrokardiogramm-(ECG)-Leitungen (36, 37, 38), einschließlich einer gemeinsamen Leitung (37) zum Anschließen an den Körper des Patienten; und
einen Hilfsstromsensor, der zum Überwachen eines Stroms, welcher durch die gemeinsame Leitung (37) fließt, und zum Betätigen des Schalters zum Unterbrechen des Stromanschlusses, wenn der Strom einen vorgegebenen Grenzwert übersteigt, angeschlossen ist.

2. Apparat nach Anspruch 1, wobei die gemeinsame Leitung (37) an ein rechtes Bein des Patienten angeschlossen werden kann.

3. Apparat nach Anspruch 1, wobei der Hilfsstromsensor einen Widerstand, der zwischen der gemeinsamen Leitung und einem Referenzpunkt angeschlossen ist, und eine Abtastschaltung (70) umfasst, die zum Messen eines Spannungsabfalls über dem Widerstand angeschlossen ist.

4. Apparat nach Anspruch 1, der ferner einen Stromsensor umfasst, welcher zum Erfassen eines Stroms, der in einem Masseleiter des Netzanschlusses fließt, und zum Betätigen des Schalters zum Unterbrechen des Netzanschlusses, wenn der Strom einen vorgegebenen Schwellwert übersteigt, angeschlossen ist.

5. Apparat nach Anspruch 4, wobei die elektrische Stromversorgung (42) mehrere Stromversorgungen (50) umfasst, die parallel angeordnet sind, um die medizinische Vorrichtung mit Strom zu versorgen.

6. Apparat nach Anspruch 5, wobei die mehreren Stromversorgungen so ausgelegt sind, dass sie Hochfrequenz-(RF)-Energie an eine Elektrode an der medizinischen Vorrichtung (28) zur Ablation von Gewebe vom Körper liefern.

7. Apparat nach Anspruch 5, wobei jede der mehreren Stromversorgungen (50) jeweils einen ausreichenden Grad an Isolierung hat, so dass ein Leckstrom in dem Masseleiter auf Grund der Anlegung einer AC-Leitungsspannung an die medizinische Vorrichtung kleiner als ein Grenzwert ist, der von der Norm 60601-1 vorgesehen ist, während die Summe des Leckstroms über alle Stromversorgungen den Grenzwert übersteigt.

## Revendications

1. Appareil médical, comprenant :
une alimentation électrique (42), qui est configurée pour une connexion à un dispositif médical électrique (28) qui est couplé au corps d'un patient et qui comprend une connexion électrique ;
un commutateur (60), qui est couplé pour connecter et déconnecter la connexion électrique ;
une pluralité de dérivations d'électrocardiogramme (ECG) (36, 37, 38), comprenant une dérivation commune (37) pour un couplage au corps du patient ; et
un capteur de courant auxiliaire, qui est couplé pour contrôler un courant circulant à travers la dérivation commune (37) et pour actionner le commutateur pour déconnecter la connexion électrique lorsque le courant dépasse une limite prédéterminée.

2. Appareil selon la revendication 1, dans lequel la dérivation commune (37) peut être connectée à la jambe droite du patient.

3. Appareil selon la revendication 1, dans lequel le capteur de courant auxiliaire comprend une résistance connectée entre la dérivation commune et un point de référence, et un circuit de détection (70) couplé pour mesurer une chute de tension à travers la résistance.

4. Appareil selon la revendication 1, comprenant en outre un capteur de courant, qui est couplé pour détecter un courant circulant dans une ligne de mise à la terre de la connexion électrique, et pour activer le commutateur pour déconnecter la connexion électrique lorsque le courant dépasse un seuil prédéterminé.

5. Appareil selon la revendication 4, dans lequel l'alimentation électrique (42) comprend une pluralité d'alimentations (50) disposées en parallèle pour alimenter en électricité le dispositif médical.

6. Appareil selon la revendication 5, dans lequel la pluralité d'alimentations électriques est configurée pour délivrer une alimentation radiofréquence (RF) à une électrode sur le dispositif médical (28) pour procéder à l'ablation d'un tissu du corps.

7. Appareil selon la revendication 5, dans lequel chacune de la pluralité d'alimentations électriques (50) a individuellement un niveau d'isolation suffisant de manière à ce qu'un courant de fuite dans la ligne de mise à la terre, dû à l'application d'une tension de courant alternatif au niveau du dispositif médical, soit inférieur à une limite fournie par la norme IEC 60601-1, tandis que la somme du courant de fuite sur toutes les alimentations électriques dépasse la limite.
